# EUROPÄISCHE PATENTSCHRIFT

(11) **EP 3 153 183 B1**
(45) Veröffentlichungstag und Bekanntmachung des Hinweises auf die Patenterteilung: **28.02.2018**
(21) Anmeldenummer: 15188481.4
(22) Anmeldetag: 06.10.2015
(51) Int. Cl.: A61L 2/07, B01J 3/04

(54) **AUTOKLAV**
AUTOCLAVE
AUTOCLAVE

(43) Veröffentlichungstag der Anmeldung: 12.04.2017
(73) Patentinhaber: Karl Hugo AG, 4770 Amel (BE)
(72) Erfinder: Hugo, Stephane, 4750 Bütgenbach (BE); Hugo, Bernd, 4960 Malmedy (BE); Henzen, Gerhard, 4770 Amel (BE)
(74) Vertreter: Kohlmann, Kai

(56) Entgegenhaltungen:
- WO-A1-2014/028063
- CN-U- 202 620 285
- FR-A1- 2 796 712
- GB-A- 991 262
- GB-A- 2 055 289

## Beschreibung

Die Erfindung betrifft einen Dampfsterilisator zum Sterilisieren von Sterilisiergut durch Einwirkung eines Sterilisationsmediums nach dem Oberbegriff des Anspruchs 1.

Unter einem Dampfsterilisator, auch als Autoklav bezeichnet, wird eine Vorrichtung verstanden, in der Dampf unter Überdruck auf das Sterilisiergut einwirkt, um Sterilität zu erreichen. Die in dem Sterilisierraum vorhandene Luft wird durch den Dampf als Sterilisationsmedium verdrängt. Dampfsterilisatoren werden in der Medizin und Biologie vor allem zur Sterilisierung von Nährmedien, medizinischen Instrumenten, Operationswäsche, Tupfer und Ähnlichem verwendet.

Dampfsterilisatoren, insbesondere für den Einsatz in der Medizin und Biologie, gibt es in unterschiedlicher Größe mit einem Innenvolumen des Sterilisierraums von bis zu einigen hundert Litern und mehr. Die vorliegende Erfindung betrifft einen Dampfsterilisator mit einem quaderförmigen, zylindrischen Sterilisierraum. An mindestens einer Seite des zylinderförmigen Sterilisierraumes befindet sich üblicherweise ein Schnellverschluss, über den der Sterilisierraum chargenweise mit dem Sterilisiergut beschickbar ist.

Ein bis zum Jahr 2008 von der Firma WESA SA, Eupen, Belgien hergestellter, gattungsgemäßer Dampfsterilisator weist einen doppelwandigen Druckbehälter mit einem quaderförmigen Innen- und einem quaderförmigen Außenmantel auf, bei dem an der Außenseite des Innenmantels und der Innenseite des Außenmantels Stege angeschweißt sind, die den Innenmantel jeweils über dessen gesamten Umfang und in regelmäßigem Abstand zwischen den stirnseitig angeordneten Schnellverschlüssen umgeben. Jeder Steg besteht aus vier Stegteilen, die an den Ecken des Sterilisierraumes überlappend verschweißt sind. Im Rahmen der regelmäßigen mechanischen Gerätesicherheitsprüfung dieser Dampfsterilisatoren wurden vielfach nach einer bestimmten Anzahl von Sterilisationszyklen Risse im Bereich der in den Ecken verschweißten Stegteile festgestellt.

Um der Rissbildung zu begegnen sind Dampfsterilisatoren mit Druckbehältern bekannt geworden, bei denen die Stege zwischen Außen- und Innenmantel jeweils aus vier Flacheisen bestehen, die in den Ecken über Knotenbleche miteinander verschweißt sind. Diese Konstruktion des Druckbehälters mit acht zu verschweißenden Teilen je Steg ist aufwendig und daher teuer in der Herstellung. Darüber hinaus lässt sich auch mit der Verwendung von Knotenblechen die Rissbildung in den Ecken nicht zuverlässig vermeiden.

Ferner ist aus der FR 2 796 712 A ein doppelwandiger Dampfsterilisator bekannt, bei dem zwischen dem Innenmantel und dem Außenmantel W-förmige Abstandshalter angeordnet sind.

Ausgehend von dem Stand der Technik liegt der Erfindung die Aufgabe zugrunde, einen Dampfsterilisator zu schaffen, der bei zumindest gleichbleibender mechanischer Gerätesicherheit mit geringerem Aufwand herstellbar ist und bei dem eine Rissbildung in Bereich der Ecken des Sterilisierraumes vermieden wird.

Diese Aufgabe wird bei einem Dampfsterilisator der eingangs erwähnten Art dadurch gelöst, dass
- sich jeder den Innenmantel umgebende Steg aus vier Stegteilen zusammensetzt,
- jedes Stegteil zwei rechtwinklig zueinander angeordnete Schenkel aufweist, wobei die Schenkel jedes Stegteils einen über die gesamte Länge gleichbleibenden, rechteckigen Querschnitt aufweisen, und
- sich die Schenkel der Stegteile im Abstand zu den Ecken des Innenmantels ausschließlich entlang der ebenen Seitenflächen des Innenmantels überlappen und dort miteinander verschweißt sind.

In den besonders rissgefährdeten Eckbereichen des Sterilisierraumes sind keinerlei Verbindungsnähte mehr zwischen den Stegteilen erforderlich. Gleichwohl müssen lediglich vier und nicht acht Stegteile miteinander verschweißt werden.

Durch die verbesserte Stabilität kann bei dem erfindungsgemäßen Dampfsterilisator gegenüber einem gleichgroßen Dampfsterilisator mit Stegen aus vier Flacheisen und vier Knotenblechen die Materialstärke des Innenmantels reduziert und der Abstand zwischen den Stegen vergrößert werden. Bei einem konkreten Dampfsterilisator mit einem Innenraumvolumen von übereinstimmend bis zu 1.000 Liter kann die Materialstärke des Innenmantels von 6 mm auf 5 mm reduziert und der Abstand zwischen den Stegen von 100 mm auf 132 mm vergrößert werden, ohne Verschlechterung der mechanischen Gerätesicherheit.

Darüber hinaus wird durch die geringere Anzahl der Stegteile sowie den größeren Abstand der Stege bei im Übrigen gleichen Abmessungen des Dampfsterilisators der Aufwand für die Verschweißung und damit der Herstellungsaufwand des Dampfsterilisators erheblich reduziert.

Zugleich ist der Dampfsterilisator bei gleicher Stabilität leichter und benötigt weniger Material zur Herstellung des Druckbehälters, der insbesondere aus rostfreiem Stahlblech besteht.

Um die Produktionskosten gering zu halten, weisen die Schenkel jedes Stegteils einen über die gesamte Länge gleichbleibenden, rechteckigen Querschnitt auf. Aufgrund der einfachen Geometrie der Stegteile lassen sich diese als Laserzuschnitte aus plattenförmigen Metallblechen herstellen.

Eine weitere Stabilisierung des Dampfsterilisators wird dadurch erreicht, dass jedes Stegteil einen kurzen und einen gegenüber dem kurzen Schenkel längeren Schenkel aufweist, wobei jeweils ein kurzer und ein langer Schenkel überlappen und miteinander verschweißt sind. Die Verschweißung im Überlappungsbereich befindet sich damit außermittig, vorzugsweise vollständig in einer Hälfte einer der Seitenflächen des Innenmantels, d.h. in einem Bereich, in dem geringere Biegungen infolge von Druck- und Temperaturschwankungen und damit geringere Belastungen der Schweißnähte auftreten. Aus konstruktiven Gründen stimmen sämtliche Stege und deren Stegteile im Aufbau überein.

Über dem Umfang des Außenmantels sind mehrere Langlöcher angeordnet. Die Langlöcher umgeben den Außenmantel in mehreren Reihen, wobei der Abstand zwischen den Reihen dem Abstand der Stege entspricht. Die in jeder Reihe angeordneten Langlöcher fluchten mit dem zwischen dem Außen- und Innenmantel unterhalb der Langlöcher angeordneten Steg. Durch die in regelmäßigem Abstand in einer Reihe angeordneten Langlöcher wird der Außenmantel mit der nach oben weisenden Oberfläche des darunter liegenden Stegs verschweißt.

Zur Verdrängung der Luft durch Sattdampf weist der Dampfsterilisator mindestens einen Einlass auf. Dieser Einlass umfasst in einer vorteilhaften Ausgestaltung der Erfindung
- einen Sammler für das Sterilisationsmedium, der an der Außenseite des Außenmantels angeordnet ist, wobei der Sammler über mindestens eine Durchtrittsöffnung mit dem Zwischenraum in Verbindung steht und vorzugsweise jeder Steg mindestens eine Durchtrittsöffnung für das Sterilisationsmedium aufweist,
- und eine Durchtrittsöffnung in dem Innenmantel, die für die Einleitung des Sterilisationsmediums aus dem Zwischenraum in den Sterilisierraum geeignet ist.

In dem der Sattdampf nicht unmittelbar, sondern über den Zwischenraum in den Sterilisierraum eingeleitet wird, kommt es zu einer raschen Temperierung des doppelwandigen Sterilisierraumes. Durch die Temperierung lässt sich der Energieaufwand zur Aufrechterhaltung der notwendigen Temperatur in dem Sterilisierraum reduzieren. Um die Verteilung des Sattdampfes in Längsrichtung des Zwischenraumes trotz der umlaufenden Stege zu gewährleisten, weist jeder Steg vorzugsweise mindestens eine Durchtrittsöffnung für das Sterilisationsmedium (Sattdampf) auf. Zusätzlich oder alternativ kann der Einlass mindestens ein gegen den Zwischenraum abgedichtetes Rohr umfassen, das das Sterilisationsmedium unmittelbar in den Sterilisierraum einleitet. Der Sterilisierraum ist über einen weiteren Sammler mit einem Dampferzeuger verbunden, der mit dem Sammler für das Sterilisationsmedium (Sattdampf) verbunden ist. Dadurch wird ein Kreislauf für das Sterilisationsmedium gebildet. Der weitere Sammler ist vorzugsweise an der Unterseite des Dampfsterilisators angeordnet.

Um Kondensat nach dem Sterilisieren einer Charge von Sterilisationsgütern aus dem Sterilisierraum abzulassen, umfasst der Auslass für das Kondensat mindestens ein gegen den Zwischenraum abgedichtetes Rohr, das sich vom Boden des Sterilisierraumes durch den Zwischenraum und den Außenmantel nach außen erstreckt. Einem Eindringen von Kondensat in den Zwischenraum und einer möglichen Hohlraumkorrosion wird hierdurch wirksam begegnet.

Mindestens eine, vorzugsweise beide stirnseitigen Wände des Sterilisierraumes sind jeweils Bestandteil eines Verschlusses. Insbesondere sind die Wände aus Vollmaterial hergestellt und an den Stirnseiten des Sterilisierraumes verschieblich geführt. Konstruktiv sind hierzu an den stirnseitigen, vertikalen Rändern des Sterilisierraumes Führungen angeordnet, in denen die Wand verschiebbar geführt ist. Jede Führung weist einen insbesondere U-förmigen Querschnitt auf, in den die seitlichen Ränder der stirnseitigen Wände eingreifen. Um den Sterilisierraum hermetisch gegenüber der Umgebung abzudichten, umgibt die stirnseitige Öffnung des Innenmantels eine umlaufende Dichtung, die an der stirnseitigen Wand zur Anlage gelangt.

Nachfolgend wird der erfindungsgemäße Dampfsterilisator anhand der Zeichnungen näher erläutert. Es zeigen
- **Figur 1**: eine perspektivische Darstellung des Sterilisierraumes mit teilweise weggebrochenem Außenmantel und ohne stirnseitige Wände,
- **Figur 1a**: eine vergrößerte Darstellung des Details H nach Figur 1,
- **Figur 2**: eine perspektivische Darstellung eines erfindungsgemäßen Dampfsterilisators,
- **Figur 2a**: eine Darstellung des Details C nach Figur 2,
- **Figur 3**: eine weitere perspektivische Darstellung des Dampfsterilisators nach Figur 2,
- **Figur 3a**: eine Darstellung des Details D nach Figur 3,
- **Figur 4**: eine Seitenansicht des Sterilisierraumes nach Figur 1,
- **Figur 4a**: einen Schnitt entlang der Linie D-D nach Figur 4 sowie
- **Figur 4b**: eine Darstellung des Details F nach Figur 4a.

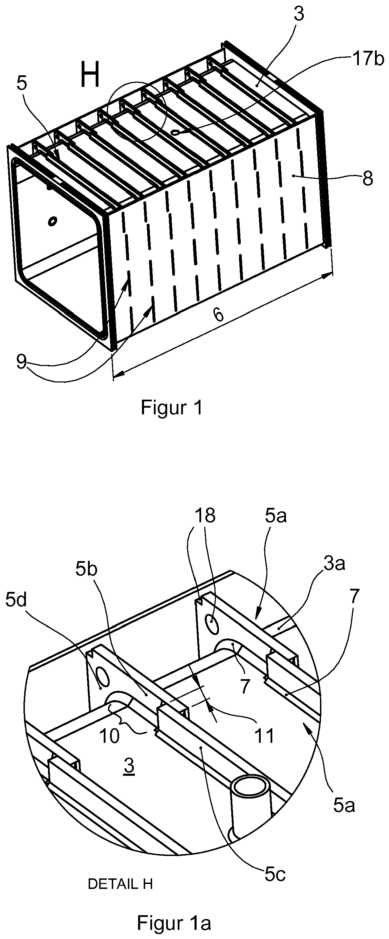

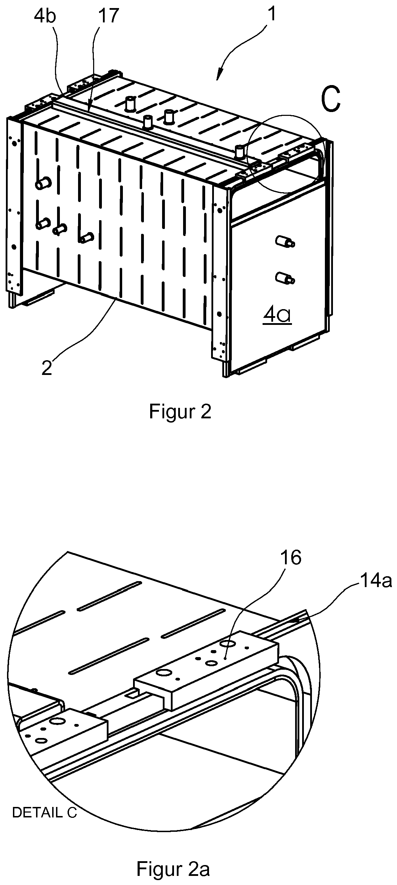

Figur 2 zeigt einen Dampfsterilisator (1) zum Sterilisieren von Sterilisiergut, wie beispielsweise medizinischen Geräten, durch Einwirkung eines Sterilisationsmediums, nämlich Sattdampf. Der Dampfsterilisator (1) umfasst einen quaderförmigen Sterilisierraum (2), der - wie in Verbindung mit Figur 1 erkennbar- durch einen Innenmantel (3) sowie zwei rechteckige, stirnseitige Wände (4a,4b) begrenzt wird. Mit der Außenseite des Innenmantels (3) sind in dem dargestellten Ausführungsbeispiel neun Stege (5) verschweißt, die den Innenmantel (3) nach Art eines Rahmens über dessen gesamten Umfang rechtwinklig zur Längsrichtung (6) des Sterilisierraums (2) umgeben. Die Stege (5) sind sämtlich übereinstimmend aufgebaut und in regelmäßigem Abstand in Längsrichtung (6) versetzt zueinander angeordnet.

Jeder Steg (5) setzt sich, wie insbesondere aus Figuren 1, 1a erkennbar, aus vier Stegteilen (5a) zusammen, wobei jedes Stegteil (5a) einen kurzen Schenkel (5b) und einen langen Schenkel (5c) aufweist, die in einem Eckbereich (5d) zusammenlaufen. Jedes Stegteil (5a) ist einteilig ausgeführt, wobei die Schenkel (5b, 5c) einen über die gesamte Länge gleichbleibenden, rechteckigen Querschnitt aufweisen. Die Stegteile (5a) bestehen vorzugsweise aus massivem Stahl und werden durch Laserschneiden aus einem Stahlblech ausgeschnitten. Die Stegteile (5a) sind entlang ihrer Längsränder mit der Außenseite des Innenmantels (3) verschweißt. In Figur 1 sind die Schweißraupen mit der Position 7 bezeichnet.

Den Innenmantel (3) umgibt ein ebenfalls quaderförmiger Außenmantel (8), der mit den nach oben weisenden Oberflächen der Stegteile (5a) verschweißt ist. Um den aus vier Deckblechen zusammengesetzten Außenmantel (8) mit den Stegteilen (5a) zu verschweißen sind über den Umfang des Außenmantels (8) insgesamt neun Reihen mit Langlöchern (9) angeordnet, die mit dem Verlauf der Stege (5) fluchten. Durch die Langlöcher (9) hindurch wird der Außenmantel (8) mit den Stegen (5) verschweißt.

Aus Figur 1a ist erkennbar, dass sich die Schenkel (5b,5c) der Stegteile (5a) im Abstand (10) zu den Ecken (3a) des Innenmantels (3) ausschließlich entlang einer der ebenen Seitenflächen des Innenmantels (3) in einem Überlappungsbereich (11) auf einer Länge von etwa 20 mm überlappen und nur dort miteinander verschweißt sind. Die Stegteile (5a) sind derart angeordnet, dass jeweils ein kurzer Schenkel (5b) einen langen Schenkel (5c) eines benachbarten Stegteils überlappt. Infolgedessen befindet sich der Überlappungsbereich (11) in einer Hälfte einer der ebenen Seitenfläche des Innenmantels (3), also in einem Bereich mit relativ geringer Verformung der Seitenfläche. Infolgedessen sind die auf die Verschweißung wirkenden Spannungen in dem Überlappungsbereich (11) gering.

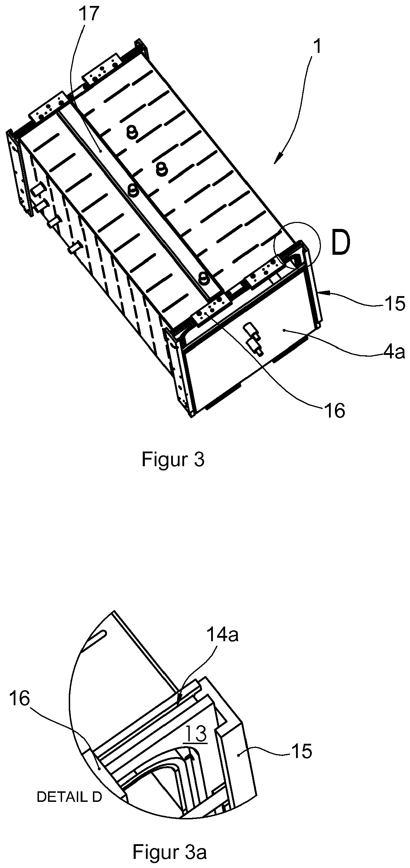

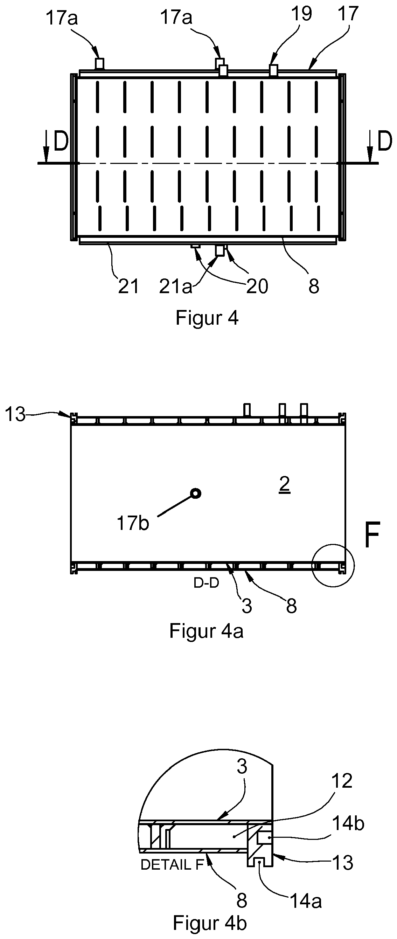

Wie insbesondere aus Figur 4b erkennbar ist der Zwischenraum (12) zwischen dem Innenmantel (3) und dem Außenmantel (8) stirnseitig durch ein Rechteckprofil (13) verschlossen. Das Rechteckprofil (13) weist am äußeren Umfang eine umlaufende Nut (14a) sowie eine an der Stirnseite des Rechteckprofils angeordnete umlaufende Nut (14b) auf. Die Nut (14b) nimmt eine über die Nutöffnung geringfügig vorstehende, in den Zeichnungen nicht dargestellte, umlaufende Dichtung auf. Die Nut (14a) dient der Befestigung von in Figur 3a erkennbaren, vertikalen Führungen (15). Die Führungen (15) bilden zusammen mit der stirnseitigen Fläche des Rechteckprofils (13) eine Gleitführung für die seitlichen Ränder, der den Sterilisierraum (3) seitlich begrenzenden Wände (4a,4b).

In dem oberen horizontalen Abschnitt der Nut (14a) sind Anschlagelemente (16) befestigt, die den Verschiebeweg der stirnseitigen Wände (4a,4b) nach oben begrenzen. Jede der beiden stirnseitigen Wände (4a,4b) ist damit Bestandteil eines sogenannten Schnellverschlusses, der die chargenweise Beladung des Sterilisierraumes (2) mit Sterilisiergut ermöglicht.

Um den Sattdampf in dem Sterilisierraum (2) einzubringen, verfügt der Dampfsterilisator (1) über einen Sammler (17) für den Sattdampf, der an der oberen Seitenfläche des Außenmantels (8) angebracht ist. Der Sammler (17) verfügt über zwei Anschlussstutzen (17a), die mit einer Zufuhr für Sattdampf, insbesondere einem Elektrodampferzeuger verbindbar sind. Der Innenraum des Sammlers (17) ist über mindestens eine Durchtrittsöffnung mit dem Zwischenraum (12) verbunden. Der Zwischenraum (12) wiederum ist mit dem Sterilisierraum (2) über eine Verrohrung oder Durchtrittsöffnungen (17b) mit dem Sterilisierraum (2) verbunden.

Der Sterilisierraum (2) ist über einen weiteren Sammler (21) an der Unterseite des Außenmantels (8) mit einem nicht dargestellten Dampferzeuger verbunden, der wiederum mit dem Sammler (17) an der Oberseite des Außenmantels (8) für das Sterilisationsmedium (Sattdampf) verbunden ist. Dadurch wird ein Kreislauf für das Sterilisationsmedium gebildet.

In den Eckbereichen (5d) sämtlicher Stegteile (5a) befinden sich weitere Durchtrittsöffnungen (18), die eine Zirkulation des Sattdampfes in Längsrichtung (6) innerhalb des Zwischenraumes (12) erlauben.

Ferner verfügt der Dampfsterilisator (1) über mehrere, gegen den Zwischenraum (12) abgedichtete Rohre (19), die unmittelbar in dem Sterilisierraum (2) münden. Sie dienen als Dampfeinlass in den Sterilisierraum.

An der Unterseite des Dampfsterilisators befindet sich ein Auslass für Kondensat, der im dargestellten Ausführungsbeispiel zwei gegen den Zwischenraum (12) abgedichtete Rohre (20) umfasst, die sich aus dem Sterilisierraum (2) durch den Zwischenraum (12) und den Außenmantel (8) nach außen erstrecken. Die Rohre (20) verfügen über nicht dargestellte Absperrorgane, die den Sterilisierraum während des Sterilisierens gegen den Umgebungsdruck abriegeln.

**Bezugszeichenliste**

| Nr. | Bezeichnung |
|---|---|
| 1. | Dampfsterilisator |
| 2. | Sterilisierraum |
| 3. | Innenmantel |
| 3a. | Ecke |
| 4a. | Wand |
| 4b. | Wand |
| 5. | Stege |
| 5a. | Stegteil |
| 5b. | Kurzer Schenkel |
| 5c. | Langer Schenkel |
| 5d. | Eckbereich |
| 6. | Längserstreckung |
| 7. | Schweißraupe |
| 8. | Außenmantel |
| 9. | Langlöcher |
| 10. | Abstand |
| 11. | Überlappungsbereich |
| 12. | Zwischenraum |
| 13. | Rechteckprofil |
| 14a. | Nut |
| 14b. | Nut |
| 15. | Führungen |
| 16. | Anschlagelemente |
| 17. | Sammler |
| 17a. | Anschlüsse |
| 17b. | Durchtrittsöffnung |
| 18. | Durchtrittsöffnung |
| 19. | Rohre |
| 20. | Rohre |
| 21. | Verteiler |
| 21a. | Verrohrung |

## Patentansprüche

1. Dampfsterilisator (1) zum Sterilisieren von Sterilisiergut durch Einwirkung eines Sterilisationsmediums umfassend
- einen quaderförmigen Sterilisierraum (2) zur Aufnahme des Sterilisiergutes,
- mindestens einen Verschluss geeignet zum Einbringen des Sterilisiergutes in den Sterilisierraum (2),
- eine Innenmantel (3) sowie zwei rechteckige, stirnseitige Wände (4a, b), die den Sterilisierraum (2) begrenzen,
- mit der Außenseite des Innenmantels (3) verschweißte Stege (5), die den Innenmantel (3) über dessen gesamten Umfang umgeben,
- einen den Innenmantel (3) umgebenden Außenmantel (8), der mit den Stegen (5) verschweißt ist,
- einen Zwischenraum (12) zwischen dem Innenmantel (3) und dem Außenmantel (8), der stirnseitig verschlossen ist,
- mindestens einen Einlass für das Sterilisationsmedium und mindestens einen Auslass für Kondensat,
- wobei sich jeder den Innenmantel (3) umgebende Steg (5) aus vier Stegteilen (5a) zusammensetzt,
**dadurch gekennzeichnet, dass**
- jedes Stegteil (5a) zwei rechtwinklig zueinander angeordnete Schenkel (5 b, c) aufweist, wobei die Schenkel (5 b, c) jedes Stegteils (5a) einen über die gesamte Länge gleichbleibenden, rechteckigen Querschnitt aufweisen, und
- sich die Schenkel (5b, c) der Stegteile (5a) im Abstand zu den Ecken (3a) des Innenmantels (3) ausschließlich entlang der ebenen Seitenflächen des Innenmantels (3) überlappen und dort miteinander verschweißt sind.

2. Dampfsterilisator nach Anspruch 1, **dadurch gekennzeichnet, dass** jedes Stegteil (5a) einen kurzen und einen gegenüber dem kurzen Schenkel (5b) längeren Schenkel (5c) aufweist, wobei jeweils ein kurzer und ein langer Schenkel (5b,c) überlappen und miteinander verschweißt sind.

3. Dampfsterilisator nach Anspruch 1 oder 2, **dadurch gekennzeichnet, dass** in Längsrichtung (6) des Innenmantels (3) mehrere Stege (5) in regelmäßigem Abstand mit der Außenseite des Innenmantels (3) verschweißt sind.

4. Dampfsterilisator nach einem der Ansprüche 1 bis 3, **dadurch gekennzeichnet, dass** sämtliche Stege (5) im Aufbau übereinstimmen.

5. Dampfsterilisator nach einem der Ansprüche 1 bis 4, **dadurch gekennzeichnet, dass** über den Umfang des Außenmantels (8) mehrere Langlöcher (9) angeordnet sind, die mit dem Verlauf der zwischen Außen- und Innenmantel (3, 8) angeordneten Stege (5) fluchten und die Stege (5) durch die Langlöcher (9) hindurch mit dem Außenmantel (8) verschweißt sind.

6. Dampfsterilisator nach einem der Ansprüche 1 bis 5, **dadurch gekennzeichnet, dass** der Einlass für das Sterilisationsmedium umfasst:
- einen Sammler (17) für das Sterilisationsmedium, der an der Außenseite des Außenmantels (8) angeordnet ist,
- wobei der Sammler (17) über mindestens eine Durchtrittsöffnung mit dem Zwischenraum (12) in Verbindung steht,
- wobei jeder Steg (5) mindestens eine Durchtrittsöffnung (18) für das Sterilisationsmedium aufweist,
- und eine Durchtrittsöffnung (17b) in dem Innenmantel (3), die für die Einleitung des Sterilisationsmediums aus dem Zwischenraum (12) in den Sterilisierraum (2) geeignet ist.

7. Dampfsterilisator nach einem der Ansprüche 1 bis 5, **dadurch grekennzeichnet,** dass der Einlass mindestens ein gegen den Zwischenraum (12) abgedichtetes Rohr (19) umfasst, das unmittelbar in dem Sterilisierraum (2) mündet.

8. Dampfsterilisator nach einem der Ansprüche 1 bis 7, **dadurch gekennzeichnet, dass** der Auslass für das Kondensat mindestens ein gegen den Zwischenraum (12) abgedichtetes Rohr (20) umfasst, das sich von der Unterseite des Sterilisierraums (2) durch den Zwischenraum (12) und den Außenmantel (6) nach außen erstreckt.

9. Dampfsterilisator nach einem der Ansprüche 1 bis 8, **dadurch gekennzeichnet, dass** mindestens eine der beiden stirnseitigen Wände (4a, b) Bestandteil eines Verschlusses ist.

10. Dampfsterilisator nach Anspruch 9, **dadurch gekennzeichnet, dass** die Wand (4a, b) verschiebbar geführt.

11. Dampfsterilisator nach Anspruch 10, **dadurch gekennzeichnet, dass** an den Stirnseiten des Dampfsterilisators (1) vertikale Führungen (15) angeordnet sind, in denen die Wand (4a, b) verschiebbar geführt ist.

12. Dampfsterilisator nach einem der Ansprüche 1 bis 11, **dadurch gekennzeichet,** dass der Zwischenraum (12) zwischen dem Innenmantel (3) und dem Außenmantel (8) an beiden Stirnseiten jeweils durch ein Rechteckprofil (13) mit einer umlaufenden Nut (14a) verschlossen ist, wobei die Nut (14a) zur Befestigung der vertikalen Führungen (15) geeignet ist.

## Claims

1. A steam steriliser (1) for sterilising material to be sterilised by the effect of a sterilisation medium, comprising
- a cuboid sterilisation chamber (2) for receiving the material to be sterilised,
- at least one closure suitable for introducing the material to be sterilised into the sterilisation chamber (2),
- an inner casing (3) and two rectangular end walls (4a, b), which delimit the sterilisation chamber (2),
- connecting pieces (5), which are welded to the outside of the inner casing (3) and surround the inner casing (3) over the entire circumference thereof,
- an outer casing (8), which surrounds the inner casing (3) and is welded to the connecting pieces (5),
- an interstice (12) between the inner casing (3) and the outer casing (8), which is closed at the end,
- at least one inlet for the sterilisation medium and at least one outlet for condensate,
- each connecting piece (5) surrounding the inner casing (3) being composed of four connecting piece parts (5a),
**characterised in that**
- each connecting piece part (5a) has two sides (5b, c) arranged at right angles to each other, wherein the sides (5b, c) of each connecting piece part (5a) have a uniform rectangular cross-section over the entire length, and
- the sides (5b, c) of the connecting piece parts (5a) overlap at a distance from the corners (3a) of the inner casing (3) only along the flat side faces of the inner casing (3) and are welded to each other there.

2. The steam steriliser according to Claim 1,
**characterised in that**
each connecting piece part (5a) has a short side (5b) and a side (5c) that is longer than the short side, wherein a short side and a long side (5b, c) overlap and are welded to each other in each case.

3. The steam steriliser according to Claim 1 or 2,
**characterised in that**
a plurality of connecting pieces (5) are welded to the outside of the inner casing (3) at regular intervals in the longitudinal direction (6) of the inner casing (3).

4. The steam steriliser according to any one of Claims 1 to 3,
**characterised in that**
all the connecting pieces (5) coincide in structure.

5. The steam steriliser according to any one of Claims 1 to 4,
**characterised in that**
a plurality of slots (9) are arranged over the circumference of the outer casing (8), said slots aligning with the profile of the connecting pieces (5) arranged between the outer and inner casings (3, 8), and the connecting pieces (5) are welded to the outer casing (8) through the slots (9).

6. The steam steriliser according to any one of Claims 1 to 5,
**characterised in that**
the inlet for the sterilisation medium comprises:
- a collector (17) for the sterilisation medium, said collector being arranged on the outside of the outer casing (8),
- wherein the collector (17) is connected to the interstice (12) via at least one through-opening,
- wherein each connecting piece (5) has at least one through-opening (18) for the sterilisation medium,
- and a through-opening (17b) in the inner casing (3), said through-opening being suitable for conducting the sterilisation medium out of the interstice (12) into the sterilisation chamber (2).

7. The steam steriliser according to any one of Claims 1 to 5,
**characterised in that**
the inlet comprises at least one tube (19), which is sealed off from the interstice (12) and opens directly into the sterilisation chamber (2).

8. The steam steriliser according to any one of Claims 1 to 7,
**characterised in that**
the outlet for the condensate comprises at least one tube (20), which is sealed off from the interstice (12) and extends outwards from the underside of the sterilisation chamber (2) through the interstice (12) and the outer casing (6).

9. The steam steriliser according to any one of Claims 1 to 8,
**characterised in that**
at least one of the two end walls (4a, b) is part of a closure.

10. The steam steriliser according to Claim 9,
**characterised in that**
the wall (4a, b) is guided in a displaceable manner.

11. The steam steriliser according to Claim 10,
**characterised in that**
vertical guides (15), in which the wall (4a, b) is guided in a displaceable manner, are arranged on the end faces of the steam steriliser (1).

12. The steam steriliser according to any one of Claims 1 to 11,
**characterised in that**
the interstice (12) between the inner casing (3) and the outer casing (8) at each end face is closed by a rectangular profile (13) with a peripheral groove (14a), wherein the groove (14a) is suitable for fastening the vertical guides (15).

## Revendications

1. Stérilisateur à vapeur (1) pour stériliser un objet à stériliser sous l'action d'un milieu stérilisant, comprenant :
- une chambre de stérilisation (2) en forme de parallélépipède, destinée à recevoir l'objet à stériliser,
- au moins une fermeture, adaptée pour l'introduction de l'objet à stériliser dans la chambre de stérilisation (2),
- une enveloppe intérieure (3) ainsi que deux parois (4a, b) frontales rectangulaires, qui délimitent la chambre de stérilisation (2),
- des entretoises (5) soudées sur la face extérieure de l'enveloppe intérieure (3), qui entourent l'enveloppe intérieure (3) sur toute sa périphérie,
- une enveloppe extérieure (8) entourant l'enveloppe intérieure (3) qui est soudée sur les entretoises (5),
- un espace intermédiaire (12) entre l'enveloppe intérieure (3) et l'enveloppe extérieure (8), qui est fermé sur la face frontale,
- au moins une entrée pour le milieu stérilisant et au moins une sortie pour du condensat,
- chacune des entretoises (5) qui entoure l'enveloppe intérieure (3) se composant de quatre parties d'entretoise (5a),
**caractérisé en ce que**
- chaque partie d'entretoise (5a) comporte deux branches (5 b, c) placées sous un angle droit mutuel, les branches (5 b, c) de chaque partie d'entretoise (5a) présentant une section transversale rectangulaire, constante sur toute la longueur et
- les branches (5b, c) des parties d'entretoise (5a) se chevauchent avec un écart par rapport aux coins (3a) de l'enveloppe intérieure (3) exclusivement le long des surfaces latérales planes (3) et y sont soudées les unes avec les autres.

2. Stérilisateur à vapeur selon la revendication 1, **caractérisé en ce que** chaque partie d'entretoise (5a) comporte une branche courte et une branche (5c) plus longue par rapport à la branche courte (5b), respectivement une branche courte et une longue (5b,c) se chevauchant et étant soudées l'une à l'autre.

3. Stérilisateur à vapeur selon la revendication 1 ou 2, **caractérisé en ce que** dans la direction longitudinale (6) de l'enveloppe intérieure (3), plusieurs entretoises (5) sont soudées avec un écart régulier sur la face extérieure de l'enveloppe intérieure (3).

4. Stérilisateur à vapeur selon l'une quelconque des revendications 1 à 3, **caractérisé en ce que** toutes les entretoises (5) coïncident dans leur structure.

5. Stérilisateur à vapeur selon l'une quelconque des revendications 1 à 4, **caractérisé en ce que** sur la périphérie de l'enveloppe extérieure (8) sont placés plusieurs trous oblongs (9} qui sont alignés sur le trajet des entretoises (5) placées entre l'enveloppe extérieure et intérieure (3, 8) et **en ce que** les entretoises (5) sont soudées sur l'enveloppe extérieure (8) à travers les trous oblongs (9).

6. Stérilisateur à vapeur selon l'une quelconque des revendications 1 à 5, **caractérisé en ce que** l'entrée pour le milieu stérilisant comprend :
- un collecteur (17) pour le milieu stérilisant qui est placé sur la face extérieure de l'enveloppe extérieure (8),
- le collecteur (17) étant en liaison avec l'espace intermédiaire (12) par l'intermédiaire d'au moins un orifice de passage,
- chaque entretoise (5) comportant au moins un orifice de passage (18) pour le milieu stérilisant,
et un orifice de passage (17b) dans l'enveloppe intérieure (3) qui est adapté pour l'introduction du milieu stérilisant à partir de l'espace intermédiaire (12) dans la chambre de stérilisation (2).

7. Stérilisateur à vapeur selon l'une quelconque des revendications 1 à 5, **caractérisé en ce que** l'entrée comprend au moins un tube (19) étanchéifié par rapport à l'espace intermédiaire (12) qui débouche directement dans la chambre de stérilisation (2).

8. Stérilisateur à vapeur selon l'une quelconque des revendications 1 à 7, **caractérisé en ce que** la sortie pour le condensat comprend au moins un tube (20) étanchéifié par rapport à l'espace intermédiaire (12) qui s'étend de la face inférieure de la chambre de stérilisation (2) vers l'extérieur, à travers l'espace intermédiaire (12) et l'enveloppe extérieure (6).

9. Stérilisateur à vapeur selon l'une quelconque des revendications 1 à 8, **caractérisé en ce qu'**au moins l'une des deux parois (4a, b) frontales est un élément d'une fermeture.

10. Stérilisateur à vapeur selon la revendication 9, **caractérisé en ce que** la paroi (4a, b) est guidée en étant translatable.

11. Stérilisateur à vapeur selon la revendication 10, **caractérisé en ce que** sur les faces frontales du stérilisateur à vapeur (1) sont placés des guidages (15) verticaux, dans lesquels la paroi (4a, b) est guidée en étant translatable.

12. Stérilisateur à vapeur selon l'une quelconque des revendications 1 à 11, **caractérisé en ce que** sur les deux faces frontales, l'espace intermédiaire (12) entre l'enveloppe intérieure (3) et l'enveloppe extérieure (8) est fermé respectivement par un profilé rectangulaire (13) avec une rainure (14a) périphérique, la rainure (14a) étant adaptée pour la fixation des guidages (15) verticaux.
